# EUROPÄISCHE PATENTSCHRIFT

(11) **EP 3 405 146 B1**
(45) Veröffentlichungstag und Bekanntmachung des Hinweises auf die Patenterteilung: **04.11.2020**
(21) Anmeldenummer: 17705074.7
(22) Anmeldetag: 09.02.2017
(51) Int. Cl.: A61F 2/915, A61F 2/954, A61F 2/82, A61F 2/06

(54) **STENT ZUM EINSATZ AN BIFURKATIONEN**
STENT FOR USING IN BIFURCATIONS
DISPOSITIF FORMANT ENDOPROTHÈSE DESTINÉ AUX BIFURCATIONS

(30) Priorität: 09.03.2016 DE 102016104302
(43) Veröffentlichungstag der Anmeldung: 28.11.2018
(73) Patentinhaber: optimed medizinische Instrumente GmbH, 76275 Ettlingen (DE)
(72) Erfinder: NENNIG, Ernst, 76131 Karlsruhe (DE); FISCHER, Harald, 76356 Weingarten (DE)
(74) Vertreter: Manitz Finsterwald Patent- und Rechtsanwaltspartnerschaft mbB
(86) Internationale Anmeldenummer: PCT/EP2017/052847
(87) Internationale Veröffentlichungsnummer: WO 2017/153116

(56) Entgegenhaltungen:
- US-A- 5 893 887
- US-A1- 2012 116 500
- US-A1- 2015 081 007
- US-B1- 6 264 686

## Beschreibung

Die vorliegende Erfindung betrifft einen Stent zur transluminalen Implantation in Hohlorgane, insbesondere in Blutgefäße, Harnleiter, Speiseröhren, Colon, Duodenum oder Gallenwege, umfassend einen ersten Stentabschnitt mit einem im Wesentlichen röhrenförmigen ersten Körper, der sich entlang einer ersten Längsachse erstreckt und ein erstes laterales Ende sowie ein erstes zentrales Ende aufweist, wobei das erste zentrale Ende des ersten Stentabschnitts abgeschrägt ausgebildet ist. Der Stent umfasst weiterhin einen zweiten Stentabschnitt mit einem im Wesentlichen röhrenförmigen zweiten Körper, der sich entlang einer zweiten Längsachse erstreckt und ein zweites laterales Ende sowie ein zweites zentrales Ende aufweist. Dabei sind der erste Stentabschnitt und der zweite Stentabschnitt im Bereich der zentralen Enden mittels eines Koppelabschnitts miteinander verbunden und der Stent umfasst eine Vielzahl von Zellen, welche von durch die röhrenförmigen Körper gebildeten Umrandungselementen definiert werden, wobei die röhrenförmigen Körper jeweils von einem komprimierten Zustand mit einem ersten Querschnittsdurchmesser in einen expandierten Zustand mit einem vergrößerten zweiten Querschnittsdurchmesser überführbar sind.

Stents dieser Art werden zur Rekanalisation von krankhaft veränderten Hohlorganen eingesetzt, wobei der Einsatz insbesondere an Abzweigungen, d.h. an Bifurkationen, erfolgt. Beispielsweise kann der erste Stentabschnitt in einer Abzweigung eines Hohlorgans mit geringerem Durchmesser eingesetzt sein, wohingegen der zweite Stentabschnitt in einem Lumen des Hohlorgans mit größerem Durchmesser angeordnet sein kann.

Zum Einsetzen des Stents werden die Stentabschnitte im komprimierten Zustand über einen gemeinsamen Einführkatheter an die zu behandelnde Stelle innerhalb des Hohlorgans eingeführt, wo sie durch unterschiedliche Maßnahmen auf einen Durchmesser, der dem Durchmesser des gesunden Hohlorgans entspricht, expandiert werden, so dass eine Stützwirkung des Hohlorgans, beispielsweise einer Gefäßwand, erreicht wird.

Derartige Stents können z.B. dadurch erzeugt werden, dass in die Wand eines röhrenförmigen Körpers Durchbrechungen, wie z.B. Schlitze, geschnitten werden, die sich teilweise in Längsrichtung des Stents erstrecken, so dass bei der Expansion des Stents beispielsweise rautenförmige Durchbrechungen entstehen. Eine Durchbrechung zusammen mit ihren Umrandungselementen wird als Zelle bezeichnet.

Bei der Verwendung von Stents mit zwei Stentabschnitten an Gabelungsstellen (Bifurkationen) eines Hohlorgans kann ein Stentabschnitt in das Lumen des Hohlorgans hineinragen, was unerwünscht ist, da sich an dem überstehenden Teil des Stents leicht Ablagerungen absetzen können, die zur Verstopfung z.B. von Blutgefäßen führen können.

Ein Stent gemäß dem Oberbegriff des Anspruchs 1 ist aus der US 2012/116500 A1 bekannt.

Es ist deshalb die der Erfindung zugrunde liegende Aufgabe, einen Stent der eingangs genannten Art anzugeben, welcher eine Verstopfung an Bifurkationen unterbindet, wobei der Stent zugleich eine hohe radiale Aufstellkraft bereitstellen soll, um das Einknicken des Stents bei dessen Einsatz zuverlässig zu verhindern.

Diese Aufgabe wird erfindungsgemäß durch einen Stent mit den Merkmalen des Anspruchs 1 und insbesondere dadurch gelöst, dass ein Teil der Zellen des ersten Stentabschnitts im Vergleich zu den übrigen Zellen des ersten Stentabschnitts in der Richtung der ersten Längsachse verlängert ausgebildet ist, um das abgeschrägte erste zentrale Ende zu bilden.

Durch das abgeschrägte erste zentrale Ende wird es ermöglicht, dass sich das erste zentrale Ende an die Form der Bifurkation des Hohlorgans anpasst, insbesondere wenn die Bifurkation schräg zu einem Hauptlumen des Hohlorgans angeordnet ist. Durch die Abschrägung wird also der Teil des ersten Stentabschnitts eingespart, der beim Einsatz an einer Bifurkation herkömmlicherweise in das Lumen des Hohlorgans hineinragen würde. Bei dem erfindungsgemäßen Stent existieren üblicherweise also keine Teile des Stents, die in das Lumen des Hohlorgans hineinragen. Auf diese Weise wird die Bildung von Ablagerungen oder gar die Verstopfung des Hohlorgans durch Ablagerungen oder Gerinselbildung unterbunden. Aufgrund der zwei Stentabschnitte können durch das Einführen nur eines Stents zwei Abschnitte der Bifurkation abgestützt werden.

Allgemein gesagt, erlaubt es der abgeschrägte Bereich, das Hohlorgan bis zur Bifurkation zuverlässig abzustützen, ohne jedoch zum Beispiel wesentlich in die Blutbahn nach der Bifurkation hineinzuragen.

Eine Zelle kann durch einen Verbindungsabschnitt oder mehrere Verbindungsabschnitte mit einer oder mehreren anderen Zellen verbunden sein. Wie erwähnt, kann eine Zelle die genannte Aussparung sowie ihre jeweiligen Umrandungselemente umfassen, wobei die Verbindungsabschnitte zu den Umrandungselementen gehören.

Bei einem erfindungsgemäßen Stent können die Zellen zumindest zum Teil jeweils mittels mehrerer Verbindungsabschnitte miteinander verbunden sein. Insbesondere können jeweils drei oder vier Verbindungsabschnitte vorgesehen sein, wodurch eine radiale Aufstellkraft des Stents und damit seine Stützwirkung besonders hoch sein können.

Der Stent kann aus einem Memory-Metall gefertigt sein, das ab einer Grenztemperatur eine gespeicherte Form einnimmt. Hierzu kann der Stent beispielsweise aus Nitinol gefertigt sein.

Die Stentabschnitte des erfindungsgemäßen Stents können durch zwei ursprünglich separate Stents gebildet sein, die in dem Koppelabschnitt miteinander verbunden, insbesondere verschweißt, sind.

Der erste und der zweite Stentabschnitt weisen jeweils zwei Enden auf, wobei die einander zugewandten Enden als zentrale Enden und die einander abgewandten Enden als laterale Enden bezeichnet werden.

Die Verbindung mittels des Koppelabschnitts kann derart ausgebildet sein, dass die Stentabschnitte zueinander beweglich ausgebildet sind und insbesondere zueinander um den Koppelabschnitt schwenkbar sind. Aufgrund der Schwenkbarkeit kann es möglich sein, die erste Längsachse des ersten Stentabschnitts parallel zu der zweiten Längsachse des zweiten Stentabschnitts anzuordnen. In einer derartigen Anordnung mit parallelen Längsachsen können die zentralen Enden einander zugewandt sein.

Beim Einsatz des erfindungsgemäßen Stents in einem Hohlorgan, beispielsweise an der Mündung der beiden Venae Iliaca Communis in die Vena Cava Inferior, insbesondere im oberen Bereich der Venae Iliaca Communis, kann die Ausrichtung der Längsachsen der Stentabschnitte verändert werden, um die Längsachsen an die Verlaufsrichtung der jeweils den Stentabschnitt beherbergenden Vene anzupassen. Hierzu können die Stentabschnitte um den Koppelabschnitt geschwenkt werden. Beispielsweise können die erste Längsachse und die zweite Längsachse im eingesetzten Zustand einen Winkel von etwa 0° bis 70° zueinander aufweisen. Die Abschrägung des ersten zentralen Endes des ersten Stentabschnitts kann einen Winkel von beispielsweise 10°, 15°, 25°, 35°, 45° oder 60° umfassen, sodass der erste Stentabschnitt - je nach Anwendungsfall - nicht in die Bifurkation hineinragt, sondern das Hohlorgan (d.h. z.B. die Vena Iliaca Communis) bis zur Bifurkation abstützt. Die genannten Winkel können dabei zwischen den Normalen der (unten erläuterten) Endebenen des ersten Stentabschnitts vorliegen.

Im oben gewählten Beispiel kann der erste Stentabschnitt beispielsweise in einer der Venae Iliaca Communis liegen, wohingegen der zweite Stentabschnitt in der Vena Cava Inferior angeordnet wird.

Aufgrund der verlängerten Zellen wird ein abgeschrägtes Ende erzeugt. Dabei werden wegen der in Richtung der ersten Längsachse verlängerten Zellen keine zusätzlichen Zellen benötigt, um das abgeschrägte Ende zu bilden. Mittels der verlängerten Zellen wird es so ermöglicht, auch in dem abgeschrägten Bereich eine ähnliche Anordnung der Zellen zu wählen, wie in dem übrigen röhrenförmigen Körper des ersten Stentabschnitts.

Die verlängerten Zellen können insbesondere nur in einem starren Abschnitt des ersten Stentabschnitts vorliegen. Zusätzlich kann der erste Stentabschnitt zum Beispiel einen flexiblen Abschnitt und/oder einen Verankerungsabschnitt umfassen. Die nachfolgenden Ausführungen bezüglich der verlängerten Zellen beziehen sich auf den starren Abschnitt.

Durch die Vermeidung zusätzlicher Zellen kann sich eine Struktur des ersten Stentabschnitts ergeben, die eine besonders hohe radiale Aufstellkraft bereitstellen kann. Auf diese Weise wird es ermöglicht, beispielsweise Blutgefäße in der Nähe von Bifurkationen zuverlässig abzustützen.

Weiterhin erlaubt es der Verzicht auf zusätzliche Zellen, den Winkel der Abschrägung variabel festzulegen, da dieser Winkel durch die relative Verlängerung der verlängerten Zellen bei der Herstellung des Stents festgelegt werden kann.

Bei den verlängerten Zellen können die Verbindungsabschnitte zwischen den Zellen (das heißt zwischen den verlängerten Zellen) ebenfalls verlängert ausgebildet sein.

Bevorzugte Ausführungsformen der Erfindung sind der Beschreibung, den Unteransprüchen und den Zeichnungen zu entnehmen.

Gemäß einer ersten vorteilhaften Ausführungsform definieren die zentralen und die lateralen Enden zentrale und laterale Endebenen, wobei die lateralen Endebenen parallel zueinander sind, wenn die erste Längsachse und die zweite Längsachse parallel zueinander sind.

Zusätzlich kann auch die zweite zentrale Endebene parallel zu der zweiten lateralen Endebene sein.

Alternativ ist es auch möglich, dass neben dem ersten zentralen Ende auch das zweite zentrale Ende und/oder das erste laterale Ende und/oder das zweite laterale Ende abgeschrägt ausgebildet ist. Hierdurch können zusätzliche Einsatzgebiete für den Stent erschlossen werden, beispielsweise in Bereichen zwischen zwei Bifurkationen.

Die Enden der Stentabschnitte können jeweils Endebenen definieren, das heißt, eine virtuelle Fläche darstellen, die durch die in Richtung der Längsachse jeweils letzten Umrandungselemente des Stentabschnitts definiert wird. Aufgrund der Abschrägung können die zentralen Endebenen einen Winkel zueinander aufweisen.

Anders ausgedrückt kann ein Normalenvektor der Endebene des ersten zentralen Endes einen Winkel mit der Längsachse einschließen. Dieser Winkel kann beispielsweise im Bereich zwischen 10° und 60°, bevorzugt zwischen 30° und 40°, liegen.

Hierbei wird die Endebene des ersten zentralen Endes als erste zentrale Endebene, die Endebene des zweiten zentralen Endes als zweite zentrale Endebene, die Endebene des ersten lateralen Endes als erste laterale Endebene und die Endebene des zweiten lateralen Endes als zweite laterale Endebene bezeichnet.

Gemäß einer weiteren vorteilhaften Ausführungsform enthält die erste zentrale Endebene des ersten zentralen Endes den Koppelabschnitt und weist mit steigender Entfernung zu dem Koppelabschnitt einen abnehmenden Abstand zu der ersten lateralen Endebene des ersten lateralen Endes auf. Anders ausgedrückt kann die Abschrägung des ersten zentralen Endes derart angeordnet sein, dass der erste Stentabschnitt dort die größte Längserstreckung in Richtung der ersten Längsachse aufweist, wo der erste Stentabschnitt in den Koppelabschnitt übergeht. Die erste zentrale Endebene kann sich also zu der ersten lateralen Endebene hin neigen, wohingegen sich die erste zentrale Endebene von der zweiten lateralen Endebene weg neigt, wenn die Längsachsen parallel zueinander sind.

Gemäß einer weiteren vorteilhaften Ausführungsform sind der erste Stentabschnitt und der zweite Stentabschnitt jeweils nur an einer Längsseite miteinander verbunden und zueinander schwenkbar ausgebildet. Unter der Längsseite ist der Bereich des jeweiligen Stentabschnitts zu verstehen, der in Richtung der jeweiligen Längsachse die längste Ausdehnung besitzt.

Der Koppelabschnitt kann eine Scharnierfunktion erfüllen, um die Schwenkbarkeit der Stentabschnitte zueinander zu gewährleisten. Hierbei können die Stentabschnitte schwenkbar um den Koppelabschnitt sein.

Gemäß einer weiteren vorteilhaften Ausführungsform sind der erste Stentabschnitt und der zweite Stentabschnitt jeweils an mehreren, bevorzugt genau an jeweils zwei, benachbarten oder aneinander angrenzenden Zellen mit dem jeweils anderen Stentabschnitt verbunden. Der Koppelabschnitt kann dabei die verbundenen Zellen umfassen. Durch die Verbindung mittels zweier Zellen kann eine stabile Verbindung der Stentabschnitte geschaffen werden, wobei trotzdem eine Verschwenkbarkeit der Stentabschnitte zueinander erhalten bleibt. Zudem kann auf diese Weise eine flächigere Abstützung des Gewebes im Bereich des Koppelabschnitts vorgenommen werden, wodurch ein Einfallen des Gewebes vermieden werden kann.

Alternativ können der erste Stentabschnitt und der zweite Stentabschnitt an genau einer Zelle mit dem jeweils anderen Stentabschnitt verbunden sein. Durch die Verbindung lediglich an einem Punkt kann eine vergrößerte "Beweglichkeit" der Stentabschnitte zueinander erreicht werden, da die Stentabschnitte dann in gewissem Umfang gegeneinander rotiert werden können.

Bevorzugt kann der Stent einstückig ausgebildet sein. Dies bedeutet, dass die Stentabschnitte z.B. aus einem Stück eines gemeinsamen Ausgangsmaterials geformt sind. Die Stentabschnitte können dementsprechend aus demselben Material gebildet sein.

Gemäß einer weiteren vorteilhaften Ausführungsform ist an jedem Ende zumindest ein Marker vorgesehen, der insbesondere die Form einer Öse aufweist, in die ein Plättchen aus röntgendichtem Material, wie z. B. Tantal, eingesetzt ist. Der Marker kann dazu dienen, die Sichtbarkeit des Stents im Röntgenbild zu erhöhen, so dass die Position des Stents im Röntgenbild gut erkennbar ist. Hierzu kann der Marker allgemein ein Abschnitt des Stents sein, der eine erhöhte Röntgendichtigkeit aufweist, wodurch er in einem Röntgenbild besonders gut sichtbar ist.

Aufgrund der Anbringung eines Markers an jedem Ende der Stentabschnitte sind die beiden Stentabschnitte im Röntgenbild gut voneinander zu unterscheiden und in ihrer Ausrichtung erkennbar.

Bevorzugt kann der Koppelabschnitt zumindest einen Marker umfassen, der insbesondere die Form einer Öse aufweist. Beispielsweise kann der Koppelabschnitt genau zwei Marker umfassen, die jeweils eine z.B. mit Tantal, Silber, Niob, Wolfram und/oder Molybdän gefüllte oder bedeckte Öse sind. Der Koppelabschnitt kann folglich durch einen, zwei oder mehrere Marker gebildet werden, wobei sich die zentralen Enden die Marker des Koppelabschnitts "teilen" können.

Gemäß einer weiteren vorteilhaften Ausführungsform ist im expandierten Zustand der Querschnittsdurchmesser des ersten Stentabschnitts kleiner als der Querschnittsdurchmesser des zweiten Stentabschnitts. Der zweite Stentabschnitt eignet sich daher zum Abstützen größerer Lumen, wohingegen der erste Stentabschnitt für Lumen mit kleinerem Durchmesser geeignet ist. Die Querschnittsdurchmesser der Stentabschnitte können auch gleich sein. Grundsätzlich können beliebige Kombinationen von Querschnittsdurchmessern der Stentabschnitte gewählt werden, wobei der Querschnittsdurchmesser des zweiten Stentabschnitts auch kleiner sein kann, als der Querschnittsdurchmesser des ersten Stentabschnitts.

Der erste und der zweite Stentabschnitt können Querschnittsdurchmesser von größer oder gleich 12 mm aufweisen. Bevorzugt kann der erste Stentabschnitt einen Querschnittsdurchmesser von 12 mm und der zweite Stentabschnitt einen Querschnittsdurchmesser von 18 mm besitzen.

Insbesondere aufgrund der unterschiedlichen Querschnittsdurchmesser können die Längsachsen bei paralleler Anordnung gegeneinander versetzt angeordnet sein. Weist beispielsweise der erste Stentabschnitt einen kleineren Querschnittsdurchmesser auf als der zweite Stentabschnitt, ergibt sich die Möglichkeit, benachbart zu dem ersten Stentabschnitt einen Zusatzstent anzuordnen, der an den zweiten Stentabschnitt angrenzt oder in den zweiten Stentabschnitt hineinragt. Der Zusatzstent kann dazu dienen, beide Abzweigungen einer Bifurkation abzustützen. Der Zusatzstent kann ebenfalls ein abgeschrägtes Ende aufweisen. Zur einfacheren Handhabung des Zusatzstents kann dieser unabhängig von den beiden Stentabschnitten sein und nicht dauerhaft an den Stentabschnitten befestigt sein.

Gemäß einer weiteren vorteilhaften Ausführungsform ist zumindest ein Teil der verlängerten Zellen entlang einer geraden oder annähernd geraden Linie angeordnet, die insbesondere parallel oder annähernd parallel zu der ersten Längsachse verläuft.

Zusätzlich oder alternativ können die verlängerten Zellen in mehrere Gruppen, insbesondere in neun Gruppen oder in 13 Gruppen, einteilbar sein, wobei die Zellen jeder Gruppe jeweils entlang einer geraden oder annähernd geraden Linie angeordnet sind, wobei die Linien insbesondere parallel oder annähernd parallel zu der ersten Längsachse verlaufen.

Insgesamt können bei dem Stent zwölf oder 16 Gruppen von Zellen vorgesehen sein, von welchen neun bzw. 13 Gruppen verlängerte Zellen aufweisen.

Die Anordnung der Zellen kann derart gewählt sein, dass die jeweiligen Zellen entlang von geraden oder annähernd geraden Linien angeordnet sind. Zusätzlich können die Zellen symmetrisch zu einer dieser Linien ausgebildet sein. Es können insbesondere keine Zellen vorliegen, die geneigt bzw. gedreht zu den übrigen Zellen sind. Auf diese Weise kann eine Schwächung der Struktur durch solche gedrehten Zellen vermieden werden. Insbesondere können alle Zellen innerhalb einer Gruppe in Längsrichtung gesehen jeweils die gleiche oder die annähernd gleiche Länge aufweisen. Alternativ können in einer Gruppe auch Zellen mit unterschiedlichen Längen vorgesehen sein.

Bevorzugt verlaufen die Linien, welche durch die Zellen verschiedener Gruppen des ersten Stentabschnitts gebildet werden, parallel oder annähernd parallel zueinander. Zudem können die Linien parallel oder annähernd parallel zu der ersten Längsachse verlaufen.

Gemäß einer weiteren vorteilhaften Ausführungsform sind von einer sich senkrecht zur ersten Längsrichtung erstreckenden Querschnittsebene bis zu dem abgeschrägten ersten zentralen Ende jeweils gleich viele Zellen in jeder Gruppe vorgesehen. Durch das Vorsehen jeweils gleich vieler Zellen in einer Gruppe kann eine radiale Aufstellkraft bewirkt werden, welche über die Länge des ersten Stentabschnitts im Wesentlichen konstant ist. Bevorzugt können in dem starren Abschnitt des ersten Stentabschnitts in jeder Gruppe gleich viele Zellen vorgesehen sein.

Alternativ oder zusätzlich kann die Länge der Zelle benachbarter Gruppen in Umfangsrichtung von einem Maximum bis zu einem Minimum fallen. Insbesondere liegen Zellen mit maximaler Länge Zellen mit minimaler Länge bezüglich der ersten Längsachse des Stents gegenüber. Durch eine derartige Anordnung kann die Abschrägung erzeugt werden.

Gemäß einer weiteren vorteilhaften Ausführungsform umfasst der erste Stentabschnitt im Bereich des ersten lateralen Endes einen flexiblen Abschnitt. Der flexible Abschnitt kann Zellen aufweisen, welche eine größere Fläche aufweisen als Zellen des restlichen ersten Stentabschnitts. Aufgrund der größeren Zellen kann der flexible Abschnitt leichter biegbar sein, wodurch sich der flexible Abschnitt einfacher an die Verlaufsform eines Hohlorgans anpassen kann. Bevorzugt können die Zellen des flexiblen Abschnitts eine zahnförmige Begrenzung aufweisen.

Gemäß noch einer weiteren vorteilhaften Ausführungsform umfasst der erste Stentabschnitt einen Verankerungsabschnitt, der sich an den flexiblen Abschnitt anschließt. Die Zellen des Verankerungsabschnitts können den Zellen des starren Abschnitts entsprechen und beispielsweise rautenförmig ausgebildet sein. Der Verankerungsabschnitt kann aufgrund der rautenförmigen Zellen eine geringere Flexibilität aufweisen und so den Stent an seiner Position in dem Hohlorgan fixieren. Der Verankerungsabschnitt kann ein gerades Ende des ersten Stentabschnitts bilden, das heißt die Endebene des ersten lateralen Endes hat die erste Längsachse als normalen Vektor. Die Marker des ersten lateralen Endes können dementsprechend an dem starren Abschnitt des ersten Stentabschnitts angebracht sein.

Nachfolgend wird die Erfindung rein beispielhaft unter Bezugnahme auf die beigefügten Zeichnungen beschrieben. Es zeigen:
- Fig. 1: einen erfindungsgemäßen Stent im expandierten Zustand in Draufsicht; und
- Fig. 2: den Stent von Fig. 1 im expandierten Zustand in einer Seitenansicht mit zueinander geschwenkten Stentabschnitten.

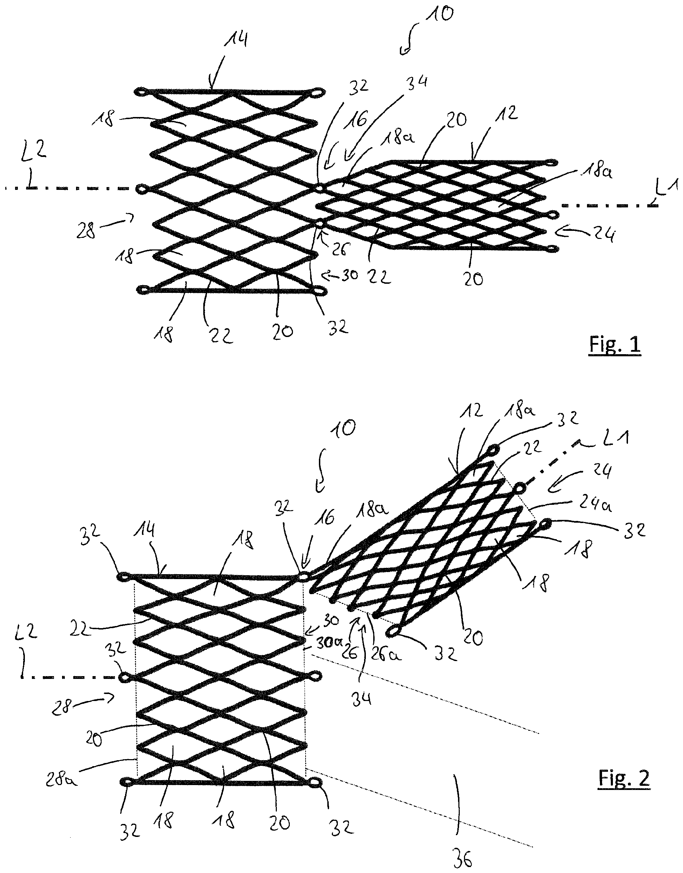

Die Fig. 1 und 2 zeigen einen Stent 10, der einen ersten Stentabschnitt 12 und einen zweiten Stentabschnitt 14 umfasst. Der erste Stentabschnitt 12 und der zweite Stentabschnitt 14 sind mittels eines Koppelabschnitts 16 miteinander verbunden.

Der erste Stentabschnitt 12 und der zweite Stentabschnitt 14 sind jeweils im Wesentlichen röhrenförmige Körper mit insbesondere kreisförmigem Querschnitt, wobei sich der erste Stentabschnitt 12 entlang einer ersten Längsachse L1 und der zweite Stentabschnitt 14 entlang einer zweiten Längsachse L2 erstrecken.

In Fig. 1 verlaufen die Längsachsen L1, L2 parallel und koaxial zueinander, wohingegen in Fig. 2 eine Anordnung des Stents gezeigt ist, bei welcher die Längsachsen L1, L2 bei Projektion auf die Zeichenebene einen Winkel von etwa 35° miteinander einschließen. Um einen solchen Winkel zu erreichen, sind die Stentabschnitte 12, 14 um den Koppelabschnitt 16 geschwenkt. Die Anordnung von Fig. 2 entspricht etwa der Ausrichtung der Stentabschnitte 12, 14 beim Einsatz des Stents 10 an einer Bifurkation.

Die Stentabschnitte 12, 14 sind aus rautenförmigen (geschlossenen) Zellen 18 gebildet, welche jeweils über drei oder vier Verbindungsabschnitte 20 mit anderen rautenförmigen Zellen 18 verbunden sind. Die rautenförmigen Zellen 18 werden durch stegartige Umrandungselemente 22 definiert, die aus einem Metall geformt sind.

Der erste Stentabschnitt 12 umfasst ein erstes laterales Ende 24 und ein erstes zentrales Ende 26. Der zweite Stentabschnitt 14 umfasst ein zweites laterales Ende 28 und ein zweites zentrales Ende 30.

Das erste laterale Ende 24 definiert eine erste laterale Endebene 24a. In entsprechender Weise definieren das erste zentrale Ende 26 eine erste zentrale Endebene 26a, das zweite laterale Ende 28 eine zweite laterale Endebene 28a und das zweite zentrale Ende 30 eine zweite laterale Endebene 30a. Zur besseren Übersicht sind die Endebenen 24a, 26a, 28a, 30a nur in Fig. 2 dargestellt.

Die zentralen Enden 26, 30 grenzen im Bereich des Koppelabschnitts 16 aneinander an, wobei eine Verbindung zwischen den Stentabschnitten 12, 14 durch zwei Ösen hergestellt wird, die als Marker 32 dienen. Jeder der Marker 32 des Koppelabschnitts 16 verbindet dabei eine Zelle 18 des ersten Stentabschnitts 12 mit einer Zelle 18 des zweiten Stentabschnitts 14.

Weitere Marker 32 sind auch an dem ersten lateralen Ende 24 und dem zweiten lateralen Ende 28 vorgesehen. Die Marker 32 sind mit Tantal (nicht gezeigt) gefüllt, um im Röntgenbild gut sichtbar zu sein. Anstelle von oder zusätzlich zu Tantal können auch Silber, Niob, Wolfram und/oder Molybdän verwendet werden.

Die Zellen 18 des zweiten Stentabschnitts 14 sind jeweils gleichförmig ausgebildet, so dass die Endebenen 28a, 30a, die durch die zweiten Enden 28, 30 des zweiten Stentabschnitts 14 definiert werden, parallel zueinander verlaufen.

Im ersten Stentabschnitt 12 sind hingegen verlängerte Zellen 18a vorgesehen, welche in Richtung der ersten Längsachse L1 verlängert ausgebildet sind. Aufgrund der verlängerten Zellen 18a wird an dem ersten zentralen Ende 26 eine Abschrägung 34 erzeugt. Zwei der verlängerten Zellen 18a sind mit den Markern 32 des Koppelabschnitts 16 verbunden.

Durch die Abschrägung 34 kann es ermöglicht werden, dass der erste Stentabschnitt 12 beim Einsatz des Stents 10 an einer Bifurkation nicht in das Lumen des abzustützenden Hohlorgans hineinragt.

Weiterhin schafft die Abschrägung 34 und die koaxiale Anordnung der Längsachsen L1, L2 die Möglichkeit einen Zusatzstent 36 in den zweiten Stentabschnitt 14 einzustecken, wie dies in Fig. 2 angedeutet ist.

### Bezugszeichenliste

- 10: Stent
- 12: erster Stentabschnitt
- 14: zweiter Stentabschnitt
- 16: Koppelabschnitt
- 18: Zelle
- 18a: verlängerte Zelle
- 20: Verbindungsabschnitt
- 22: Umrandungselement
- 24: erstes laterales Ende
- 24a: erste laterale Endebene
- 26: erstes zentrales Ende
- 26a: erste zentrale Endebene
- 28: zweites laterales Ende
- 28a: zweite laterale Endebene
- 30: zweites zentrales Ende
- 30a: zweite zentrale Endebene
- 32: Marker
- 34: Abschrägung
- 36: Zusatzstent

- L1: erste Längsachse
- L2: zweite Längsachse

## Patentansprüche

1. Stent (10) zur transluminalen Implantation in Hohlorgane, insbesondere in Blutgefäße, Harnleiter, Speiseröhren, Colon, Duodenum oder Gallenwege, umfassend
einen ersten Stentabschnitt (12) mit einem im Wesentlichen röhrenförmigen ersten Körper, der sich entlang einer ersten Längsachse (L1) erstreckt und ein erstes laterales Ende (24) sowie ein erstes zentrales Ende (26) aufweist, und
einen zweiten Stentabschnitt (14) mit einem im Wesentlichen röhrenförmigen zweiten Körper, der sich entlang einer zweiten Längsachse (L2) erstreckt und ein zweites laterales Ende (28) sowie ein zweites zentrales Ende (30) aufweist
wobei
der erste Stentabschnitt (12) und der zweite Stentabschnitt (14) im Bereich der zentralen Enden (26, 30) mittels eines Koppelabschnitts (16) miteinander verbunden sind,
der Stent (10) eine Vielzahl von Zellen (18, 18a) umfasst, welche von durch die röhrenförmigen Körper gebildeten Umrandungselementen (22) definiert werden, wobei die röhrenförmigen Körper jeweils von einem komprimierten Zustand mit einem ersten Querschnittsdurchmesser in einen expandierten Zustand mit einem vergrößerten zweiten Querschnittsdurchmesser überführbar sind, und wobei
das erste zentrale Ende (26) des ersten Stentabschnitts abgeschrägt ausgebildet ist,
**dadurch gekennzeichnet, dass**
ein Teil der Zellen (18a) des ersten Stentabschnitts im Vergleich zu den übrigen Zellen (18) des ersten Stentabschnitts (12) in der Richtung der ersten Längsachse (L1) verlängert ausgebildet ist, um das abgeschrägte erste zentrale Ende (26) zu bilden.

2. Stent (10) nach Anspruch 1,
**dadurch gekennzeichnet , dass**
die zentralen und die lateralen Enden (24, 26, 28, 30) zentrale und laterale Endebenen (24a, 26a, 28a, 30a) definieren, wobei die lateralen Endebenen (24a, 28a) parallel zueinander sind, wenn die erste Längsachse (L1) und die zweite Längsachse (L2) parallel zueinander sind.

3. Stent (10) nach Anspruch 2,
**dadurch gekennzeichnet , dass**
die erste zentrale Endebene (26a) des ersten zentralen Endes (26) den Koppelabschnitt (16) enthält und mit steigender Entfernung zu dem Koppelabschnitt (16) einen abnehmenden Abstand zu der ersten lateralen Endebene (24a) des ersten lateralen Endes (24) aufweist.

4. Stent (10) nach zumindest einem der Ansprüche 1 bis 3,
**dadurch gekennzeichnet, dass**
der erste Stentabschnitt (12) und der zweite Stentabschnitt (14) jeweils an einer Längsseite miteinander verbunden sind und zueinander schwenkbar ausgebildet sind.

5. Stent (10) nach zumindest einem der vorstehenden Ansprüche,
**dadurch gekennzeichnet , dass**
der erste Stentabschnitt (12) und der zweite Stentabschnitt (14) jeweils an mehreren, bevorzugt genau an jeweils zwei, benachbarten oder aneinander angrenzenden Zellen (18, 18a) mit dem jeweils anderen Stentabschnitt (12, 14) verbunden sind.

6. Stent (10) nach zumindest einem der Ansprüche 1 bis 4,
**dadurch gekennzeichnet, dass**
der erste Stentabschnitt (12) und der zweite Stentabschnitt (14) an jeweils genau einer Zelle (18, 18a) mit dem jeweils anderen Stentabschnitt (12, 14) verbunden sind.

7. Stent (10) nach zumindest einem der vorstehenden Ansprüche,
**dadurch gekennzeichnet, dass**
der Stent (10) einstückig ausgebildet ist.

8. Stent (10) nach zumindest einem der vorstehenden Ansprüche,
**dadurch gekennzeichnet, dass**
an jedem Ende (24, 26, 28, 30) zumindest ein Marker (32) vorgesehen ist, der insbesondere die Form einer Öse aufweist.

9. Stent (10) nach zumindest einem der vorstehenden Ansprüche,
**dadurch gekennzeichnet, dass**
der Koppelabschnitt (16) zumindest einen Marker (32) umfasst, der insbesondere die Form einer Öse aufweist.

10. Stent (10) nach zumindest einem der vorstehenden Ansprüche,
**dadurch gekennzeichnet , dass**
im expandierten Zustand der Querschnittsdurchmesser des ersten Stentabschnitts (12) kleiner ist als der Querschnittsdurchmesser des zweiten Stentabschnitts (14).

11. Stent (10) nach zumindest einem der vorstehenden Ansprüche,
**dadurch gekennzeichnet, dass**
zumindest ein Teil der verlängerten Zellen (18a) entlang einer geraden oder annähernd geraden Linie angeordnet ist, die insbesondere parallel oder annähernd parallel zu der ersten Längsachse (L1) verläuft, und/oder die verlängerten Zellen (18a) in mehrere Gruppen, insbesondere in neun Gruppen, einteilbar sind, wobei die Zellen jeder Gruppe jeweils entlang einer geraden oder annähernd geraden Linie angeordnet sind, wobei die Linien insbesondere parallel oder annähernd parallel zu der ersten Längsachse (L1) verlaufen.

12. Stent (10) nach Anspruch 11,
**dadurch gekennzeichnet, dass**
von einer sich senkrecht zur ersten Längsrichtung (L1) erstreckenden Querschnittsebene bis zu dem abgeschrägten ersten zentralen Ende (26) jeweils gleich viele Zellen (18, 18a) in jeder Gruppe vorgesehen sind und/oder
die Länge der Zellen (18, 18a) benachbarter Gruppen in Umfangsrichtung von einem Maximum bis zu einem Minimum fällt.

13. Stent (10) nach zumindest einem der vorstehenden Ansprüche,
**dadurch gekennzeichnet, dass**
der erste Stentabschnitt im Bereich des ersten lateralen Endes (24) einen flexiblen Abschnitt umfasst.

## Claims

1. A stent (10) for transluminal implantation into hollow organs, in particular into blood vessels, ureters, esophagi, the colon, the duodenum or bile ducts, comprising
a first stent section (12) having a substantially tubular first body which extends along a first longitudinal axis (L1) and which has a first lateral end (24) and a first central end (26); and
a second stent section (14) having a substantially tubular second body which extends along a second longitudinal axis (L2) and which has a second lateral end (28) and a second central end (30),
wherein
the first stent section (12) and the second stent section (14) are connected to one another by means of a coupling section (16) in the region of the central ends (26, 30); wherein
the stent (10) comprises a plurality of cells (18, 18a) which are defined by bordering elements (22) formed by the tubular bodies, with the tubular bodies each being able to be transformed from a compressed state having a first cross-section diameter into an expanded state having an increased second cross-section diameter; and wherein
the first central end (26) of the first stent section is chamfered,
**characterized in that**
some of the cells (18a) of the first stent section are elongated in the direction of the first longitudinal axis (L1) in comparison with the other cells (18) of the first stent section (12) to form the chamfered first central end (26).

2. A stent (10) in accordance with claim 1,
**characterized in that**
the central and lateral ends (24, 26, 28, 30) define central and lateral end planes (24a, 26a, 28a, 30a), with the lateral end planes (24a, 28a) being in parallel with one another when the first longitudinal axis (L1) and the second longitudinal axis (L2) are in parallel with one another.

3. A stent (10) in accordance with claim 2,
**characterized in that**
the first central end plane (26a) of the first central end (26) includes the coupling section (16) and has a decreasing distance from the first lateral end plane (24a) of the first lateral end (24) with an increasing distance from the coupling section (16).

4. A stent (10) in accordance with at least one of the claims 1 to 3,
**characterized in that**
the first stent section (12) and the second stent section (14) are each connected to one another at a longitudinal side and are pivotable with respect to one another.

5. A stent (10) in accordance with at least one of the preceding claims,
**characterized in that**
the first stent section (12) and the second stent section (14) are connected to the respective other stent section (12, 14) at a respective plurality of, preferably at exactly a respective two, cells neighboring or adjacent to one another.

6. A stent (10) in accordance with at least one of the claims 1 to 4,
**characterized in that**
the first stent section (12) and the second stent section (14) are connected to the respective other stent section (12, 14) at exactly one respective cell (18, 18a).

7. A stent (10) in accordance with at least one of the preceding claims,
**characterized in that**
the stent (10) is formed in one piece.

8. A stent (10) in accordance with at least one of the preceding claims,
**characterized in that**
at least one marker (32) which in particular has the form of an eyelet is provided at each end (24, 26, 28, 30).

9. A stent (10) in accordance with at least one of the preceding claims,
**characterized in that**
the coupling section (16) comprises at least one marker (32) which in particular has the form of an eyelet.

10. A stent (10) in accordance with at least one of the preceding claims,
**characterized in that**
the cross-section diameter of the first stent section (12) is smaller than the cross-section diameter of the second stent section (14) in the expanded state.

11. A stent (10) in accordance with at least one of the preceding claims,
**characterized in that**
at least some of the elongated cells (18a) are arranged along a straight line or an approximately straight line which in particular extends in parallel or approximately in parallel with the first longitudinal axis (L1); and/or **in that** the elongated cells (18a) are divisible into a plurality of groups, in particular into nine groups, with the cells of each group respectively being arranged along a straight line or an approximately straight line, with the lines in particular extending in parallel or approximately in parallel with the first longitudinal axis (L1).

12. A stent (10) in accordance with claim 11,
**characterized in that**
respectively the same number of cells (18, 18a) is provided in each group from a cross-sectional plane extending perpendicular to the first longitudinal direction (L1) up to the chamfered first central end (26); and/or
**in that** the length of the cells (18, 18a) of adjacent groups drops from a maximum to a minimum in the peripheral direction.

13. A stent (10) in accordance with at least one of the preceding claims,
**characterized in that**
the first stent section comprises a flexible section in the region of the first lateral end (24).

## Revendications

1. Stent (10) pour l'implantation transluminale dans des organes creux, en particulier dans les vaisseaux sanguins, les uretères, l'œsophage, le côlon, le duodénum ou les voies biliaires, comprenant
une première portion de stent (12) comprenant un premier corps sensiblement tubulaire s'étendant le long d'un premier axe longitudinal (L1) et ayant une première extrémité latérale (24) et une première extrémité centrale (26) ; et
une seconde portion de stent (14) comprenant un second corps sensiblement tubulaire s'étendant le long d'un second axe longitudinal (L2) et ayant une seconde extrémité latérale (28) et une seconde extrémité centrale (30), dans lequel
la première portion de stent (12) et la seconde portion de stent (14) sont reliées entre elles dans la région des extrémités centrales (26, 30) au moyen d'une portion de couplage (16),
le stent (10) comprend une multitude de cellules (18, 18a) définies par des éléments de bordure (22) formés par les corps tubulaires, lesdits corps tubulaires étant chacun transformables depuis un état comprimé ayant un premier diamètre de section transversale jusqu'à un état expansé ayant un second diamètre de section transversale agrandi, et
la première extrémité centrale (26) de la première portion de stent est réalisée de façon biseautée,
**caractérisé en ce que**
une partie des cellules (18a) de la première portion de stent sont réalisées de façon prolongée dans la direction du premier axe longitudinal (L1) par rapport aux cellules restantes (18) de la première portion de stent (12), afin de former la première extrémité centrale (26) biseautée.

2. Stent (10) selon la revendication 1,
**caractérisé en ce que**
les extrémités centrales et latérales (24, 26, 28, 30) définissent des plans d'extrémité centraux et latéraux (24a, 26a, 28a, 30a), les plans d'extrémité latéraux (24a, 28a) étant parallèles entre eux lorsque le premier axe longitudinal (L1) et le second axe longitudinal (L2) sont parallèles entre eux.

3. Stent (10) selon la revendication 2,
**caractérisé en ce que**
le premier plan d'extrémité central (26a) de la première extrémité centrale (26) comprend la portion de couplage (16) et présente une distance par rapport au premier plan d'extrémité latéral (24a) de la première extrémité latérale (24), qui diminue au fur et à mesure que la distance par rapport à la portion de couplage (16) augmente.

4. Stent (10) selon l'une au moins des revendications 1 à 3,
**caractérisé en ce que**
la première portion de stent (12) et la seconde portion de stent (14) sont reliées entre elles chacune sur un côté longitudinal et sont réalisées de manière à pouvoir pivoter l'une par rapport à l'autre.

5. Stent (10) selon l'une au moins des revendications précédentes,
**caractérisé en ce que**
la première portion de stent (12) et la seconde portion de stent (14) sont chacune reliées à l'autre portion de stent respective (12, 14) au niveau de plusieurs cellules, de préférence au niveau de précisément deux cellules (18, 18a) voisines ou adjacentes l'une de l'autre.

6. Stent (10) selon l'une au moins des revendications 1 à 4,
**caractérisé en ce que**
la première portion de stent (12) et la seconde portion de stent (14) sont reliées à l'autre portion de stent respective (12, 14) au niveau de précisément une cellule (18, 18a) respective.

7. Stent (10) selon l'une au moins des revendications précédentes,
**caractérisé en ce que**
le stent (10) est réalisé d'un seul tenant.

8. Stent (10) selon l'une au moins des revendications précédentes,
**caractérisé en ce que**
au moins un marqueur (32) est prévu à chaque extrémité (24, 26, 28, 30), qui présente en particulier la forme d'un œillet.

9. Stent (10) selon l'une au moins des revendications précédentes,
**caractérisé en ce que**
la portion de couplage (16) comporte au moins un marqueur (32) qui présente en particulier la forme d'un œillet.

10. Stent (10) selon l'une au moins des revendications précédentes,
**caractérisé en ce que**
à l'état expansé, le diamètre de section transversale de la première portion de stent (12) est plus petit que le diamètre de section transversale de la seconde portion de stent (14).

11. Stent (10) selon l'une au moins des revendications précédentes,
**caractérisé en ce que**
au moins une partie des cellules prolongées (18a) sont disposées le long d'une ligne droite ou approximativement droite qui s'étend en particulier parallèlement ou approximativement parallèlement au premier axe longitudinal (L1), et/ou
les cellules prolongées (18a) peuvent être subdivisées en plusieurs groupes, en particulier en neuf groupes, les cellules de chaque groupe étant disposées le long d'une ligne droite ou approximativement droite, les lignes s'étendant en particulier parallèlement ou approximativement parallèlement au premier axe longitudinal (L1).

12. Stent (10) selon la revendication 11,
**caractérisé en ce que**
depuis un plan de section transversale s'étendant perpendiculairement à la première direction longitudinale (L1) jusqu'à la première extrémité centrale biseautée (26), il est prévu le même nombre de cellules (18, 18a) dans chaque groupe, et/ou
la longueur des cellules (18, 18a) des groupes voisins tombe en direction périphérique pour passer d'un maximum à un minimum.

13. Stent (10) selon l'une au moins des revendications précédentes,
**caractérisé en ce que**
la première portion de stent comprend une portion flexible dans la région de la première extrémité latérale (24).
